# EUROPÄISCHE PATENTSCHRIFT

(11) **EP 1 596 731 B1**
(45) Veröffentlichungstag und Bekanntmachung des Hinweises auf die Patenterteilung: **13.06.2012**
(21) Anmeldenummer: 04706642.8
(22) Anmeldetag: 30.01.2004
(51) Int. Cl.: A61B 17/16

(54) **MEDIZINISCHE BOHRVORRICHTUNG**
MEDICAL DRILLING DEVICE
DISPOSITIF MEDICAL DE PERAGE

(30) Priorität: 31.01.2003 DE 10303964
(43) Veröffentlichungstag der Anmeldung: 23.11.2005
(73) Patentinhaber: Oettinger, Wolfgang, 4310 Rheinfelden (CH)
(72) Erfinder: OETTINGER, Wolfgang, 4310 Rheinfelden (CH); PARAK, Wolfgang, J., 85221 Dachau (DE)
(74) Vertreter: Hoffmann, Jörg Peter
(86) Internationale Anmeldenummer: PCT/EP2004/000886
(87) Internationale Veröffentlichungsnummer: WO 2004/066850

(56) Entgegenhaltungen:
- EP-A- 1 269 933
- DE-A- 19 954 005
- US-A- 2 763 935
- US-A- 5 785 522
- US-B1- 6 391 005

## Beschreibung

Die Erfindung betrifft eine medizinische Bohrvorrichtung zum Bohren von menschlichem oder tierischem Gewebe, mit einem Bohrantrieb zum drehenden Antreiben eines elektrisch leitenden Bohrwerkzeugs.

In der Unfallchirurgie und Orthopädie müssen bei wiederherstellenden Operationen nach Knochenbrüchen Bohrungen durch den Knochen vorgenommen werden, in die in der Regel Schrauben eingesetzt werden, deren Länge sehr präzise, auf den Millimeter genau festgelegt ist. Um die erforderliche Schraubenlänge bestimmen zu können, muss die Länge bzw. Tiefe der im Knochen erzeugten Bohrung gemessen werden, was üblicherweise unter Zuhilfenahme von Schublehren erfolgt. Dazu muss die Schublehre nach Erzeugen der Bohrung in diese eingeführt werden. Bei bestimmten Bohrungen ist es darüber hinaus erforderlich, den Durchtrittszeitpunkt des Bohrers durch den Knochen präzise zu bestimmen, wodurch gegen Ende des Bohrvorgangs das Bohren mehrfach unterbrochen werden muss, um die Schublehre in die Bohrung einzuführen. Diese Messungen sind fehlerbehaftet, behindern den Operationsablauf und verlängern die Operation insgesamt.

Im Prinzip wird ein Knochen durch zwei typische Knochenstrukturen gebildet, nämlich die Knochenrinde (Corticalis) und das Knochenmark (Spongiosa). Dabei wird das weichere Knochenmark von der harten Knochenrinde umgeben. Beim Durchbohren eines Röhrenknochens passiert der Bohrer die verschiedenen Schichten des Knochens in folgender Reihenfolge: Knochenrinde (Corticalis), Knochenmark (Spongiosa) und erneut die Knochenrinde (Gegencorticalis) am Ausgang der Bohrung. Die Gegencorticalis ist bei Operationen üblicherweise von Weichteilen (Muskulatur, Fett) umgeben, während die Knochenoberfläche am Ansatz des Bohrens, d. h. am Eingang der Bohrung, chirurgisch freigelegt wird. Dies gilt auch dann, wenn die Bohransatzstelle über Stichinzission (minimalinvasive Chirurgie) gefunden wird. In der Praxis wird die Position der Bohransatzstelle mit einer sogenannten Gewebeschutzhülse fixiert, die das Bohrwerkzeug am Eintritt in den Knochen umgibt und dadurch mit Spiel führt.

Zur Präzisierung zu der oben beschriebenen mechanischen Längenbestimmung des Bohrloches mit einer Schublehre kann es notwendig werden, den gebohrten Knochen während des Bohrens in regelmäßigen Abständen zu röntgen, was aufgrund der Strahlenbelastung nachteilig ist. Weiterhin ist es bekannt, das Bohrloch akustisch durch Ultraschall oder optisch mit Hilfe von Laserlicht zu untersuchen, wobei diese Methoden auf dem unterschiedlichen Reflexionsvermögen von Weichgewebe, Corticalis und Spongiosa basieren. Diese Methoden sind jedoch technisch aufwändig und haben - wie das Messen mit der Schublehre - den Nachteil, dass die Länge des Bohrlochs separat, d. h. bei Unterbrechung des eigentlichen Bohrvorgangs, bestimmt werden muss.

Beim Durchbohren des Knochens muss der Operateur insbesondere gegen Ende des Bohrvorgangs darauf achten, dass der Bohrer nach Durchstoßen der Gegencorticalis nicht in das dahinterliegende Weichgewebe eindringt und dort unerwünschte Verletzungen hervorruft. Zur Vermeidung derartiger Schädigungen muss der Operateur daher gegen Ende des Bohrvorgangs den Bohrvorgang gelegentlich unterbrechen, den Bohrer aus der Bohrung herausziehen und prüfen, ob die Gegencorticalis bereits durchbohrt ist.

Aus der nachveröffentlichten EP 1 474 046 AO ist eine medizinische Bohrvorrichtung mit einem elektrisch leitenden Bohrwerkzeug bekannt. Mit Hilfe einer Widerstandsmesseinrichtung ist der elektrische Widerstand des zwischen dem von einer Bohrelektrode kontaktierten Bohrwerkzeug und einer Gegenelektrode befindlichen Gewebes messbar.

Das Dokument EP-A-1 269 933, von dem sich die Präambel des Anspruchs 1 ableitet, offenbart eine gattungsgemäße medizinische Bohrvorrichtung. Bei dieser bekannten Bohrvorrichtung wird der Druck innerhalb eines Druckaufbau- und Druckmesssystems über einen Drucksensor erfasst. Fällt er unter einen vorbestimmten Schwellenwert, so schaltet ein Regler die Stromzufuhr für den Antriebsmotor der Bohrvorrichtung ab.

Der Erfindung liegt die Aufgabe zugrunde, eine medizinische Bohrvorrichtung anzugeben, mit der der Fortschritt beim Bohren durch den Operateur in einfacher Weise überwacht werden kann und die darüber hinaus eine Bestimmung der Bohrlochlänge ermöglicht.

Erfindungsgemäß wird die Aufgabe durch medizinische Bohrvorrichtungen gemäß den Ansprüchen 1. 3 oder 5 gelöst. Vorteilhafte Weiterbildungen der Erfindung sind Gegenstand der abhängigen Ansprüche.

Eine erfindungsgemäße medizinische Bohrvorrichtung weist eine Bohrelektrode, eine Gegenelektrode sowie eine mit den beiden Elektroden verbundene Widerstandsmesseinrichtung auf. Die Bohrelektrode steht mit dem elektrisch leitenden Bohrwerkzeug in elektrischem Kontakt, während die Gegenelektrode gegenüber von dem Bohrwerkzeug auf der anderen Seite des zu bohrenden Gewebes, z. B. auch auf der Außenhaut des Patienten, anbringbar ist. Mit Hilfe der Widerstandsmesseinrichtung werden die beiden Elektroden mit einem Gleich- oder Wechselstrom beaufschlagt, so dass der elektrische Widerstand zwischen dem von der Bohrelektrode kontaktierten Bohrwerkzeug und der Gegenelektrode messbar und danach auf einer Anzeigeeinrichtung anzeigbar ist.

Bei einer besonders vorteilhaften Ausführungsform der Erfindung speist die Widerstandsmesseinrichtung einen Wechselstrom ein und erfasst den Widerstand in Form einer Impedanz. Die als Impedanzmesseinrichtung dienende Widerstandsmesseinrichtung ermöglicht es, den elektrischen Widerstand des zwischen dem Bohrwerkzeug und der Gegenelektrode liegenden Gewebes besonders störungsfrei, ohne Einfluss von möglicherweise entstehenden elektrischen Feldern, statischen Aufladungen etc. zu erfassen.

Die Erfindung basiert auf der Erkenntnis, dass menschliches oder tierisches Gewebe, insbesondere also Weichgewebe, Knochenrinde und Knochenmark eine unterschiedliche elektrische Impedanz besitzen. Daher ist es durch die Impedanzmessung zwischen dem Bohrwerkzeug, insbesondere zwischen der Spitze eines Bohrers und der am Weichgewebe außen angebrachten Gegenelektrode möglich, den Aufenthaltsort der Bohrerspitze festzustellen. Insbesondere lässt sich erkennen, in welchem Gewebetyp sich der Bohrer bzw. die Bohrerspitze gerade befindet. Zum Beispiel wurde festgestellt, dass beim Durchdringen der Gegencorticalis durch die Bohrerspitze ein Impedanzsprung messbar ist, da die Bohrerspitze danach in das die Gegencorticalis umgebende Weichgewebe eindringt. Im Rückschluss lässt sich aus einem derartigen Impedanzsprung ableiten, dass die Bohrerspitze soeben die Gegencorticalis durchdrungen hat und in Kontakt mit dem Weichgewebe gelangt ist. Diese Information kann dann dem Operateur angezeigt werden, um ihn davor zu warnen, weiter zu bohren und möglicherweise intaktes Weichgewebe zu verletzen.

Besonders vorteilhaft ist es, dass die Widerstandsmessung während des eigentlichen Bohrvorgangs erfolgen kann, also ohne dass der Bohrvorgang unterbrochen werden muss.

Als elektrischer Widerstandswert lässt sich auch ein Gleichstromwiderstand ermitteln, wobei jedoch im Einzelfall zu prüfen ist, ob dieser Messwert zur Differenzierung der unterschiedlichen Gewebetypen ausreicht, oder ob Messfehler ein verwertbares Messergebnis verhindern.

Bei einer besonders vorteilhaften Ausführungsform der Erfindung ist die Anzeigeeinrichtung zum Anzeigen von optischen oder akustischen Signalen aufgrund von aus der Widerstandsmesseinrichtung gewonnenen Messwerten oder Messwertänderungen ausgebildet. Es ist dadurch möglich, dem Operateur geeignete Informationen zur Verfügung zu stellen, aufgrund denen er Rückschlüsse darauf ziehen kann, in welchem Gewebetyp gerade gebohrt wird. Die Informationen können in Form von absoluten Messwerten oder aber auch aufgrund von Messwertänderungen bereitgestellt werden. Das Erfassen von Messwertänderungen hat den Vorteil, dass sich der Operateur nur dann mit den Messergebnissen befassen muss, wenn sich an den Messwerten etwas geändert hat, d. h. wenn der Bohrer gerade in einen anderen Gewebetyp eindringt. Um den Operateur nicht von seiner Arbeit abzulenken, sind hierzu akustische Signale besonders hilfreich.

Dazu ist es vorteilhaft, wenn die Anzeigeeinrichtung eine Auswerteeinheit aufweist, mit der aufgrund der von der Widerstandsmesseinrichtung erfassten Messwerte oder Messwertänderungen Informationen über die Qualität oder den Typ des Gewebes ermittelbar sind, welches zu dem jeweiligen Zeitpunkt durch das Bohrwerkzeug gebohrt wird. Die Auswerteeinheit kann derart ausgebildet sein, dass sie eine Schalteinrichtung ansteuert, um den Bohrantrieb abzuschalten oder um ein entsprechendes Signal über die Anzeigeeinrichtung abzugeben, wenn die Auswerteeinheit das Bohren eines bestimmten Gewebetyps feststellt. Auf diese Weise ist es z. B. möglich, den Operateur gezielt darauf hinzuweisen, wenn der Bohrer nach Durchbohren des Knochens die Gegencorticalis durchstoßen hat und in Kontakt mit dem Weichgewebe gelangt. Dann nämlich sollte der Bohrvorgang sofort unterbrochen werden, um eine Schädigung des Weichgewebes zu vermeiden. Dazu könnte die Auswerteeinheit registrieren, dass die Bohrerspitze nacheinander, d. h. in dieser Reihenfolge Knochenmark und Knochenrinde gebohrt hat, und sich dann plötzlich - bei Erreichen des Weichgewebes - der Inpedanzwert ändert.

Die durch die erfindungsgemäße Bohrvorrichtung ermöglichte Feststellung, in welchem Gewebetyp jeweils aktuell gebohrt wird, erlaubt es nicht nur, den Zeitpunkt des Durchbohrens des Knochens präzise zu bestimmen. Außerdem ermöglicht es diese Information, dem Operateur bei Arbeiten in besonders schwierigen Regionen, wie z. B. in der Beckenchirurgie, den Bohrer auch in Längsrichtung durch den Knochen, und zwar durch die Spongiosa, zu führen und auf diese Weise zu verhindern, dass die Corticalis an einer nicht beabsichtigten Stelle versehentlich durchdrungen wird. Gerade in der Beckenchirurgie ist es oft erforderlich, in einem nur wenige Millimeter breiten Spongiosabereich den Bohrer zwischen den angrenzenden Corticalisschichten hindurchzuführen, ohne diese Schichten zu verletzen. Da bei einem versehentlichen Anbohren der Corticalis jeweils ein Impedanzsprung angezeigt würde, kann der Operateur gegebenenfalls den Weg des Bohrwerkzeugs korrigieren.

Das Bohrwerkzeug ist ein Bohrer aus einem elektrisch leitenden Material, der eine isolierende Ummantelung derart aufweist, dass die Bohrerspitze einerseits sowie ein ringförmiger Bereich am Bohrerschaft andererseits frei von der isolierenden Ummantelung bleiben und somit zur Umgebung hin elektrisch leitend sind. An dem ringförmigen Bereich, der vorzugsweise vor einem Bereich des Bohrerschafts angeordnet ist, an dem der Bohrer in einem Bohrfutter eingespannt wird, kann die Bohreleketrode den Bohrer kontaktieren und somit die von der Widerstandsmesseinrichtung angelegte Wechsel- oder Gleichspannung auf den Bohrer übertragen. Die Spannung bzw. der daraus resultierende Strom wird ohne messbare Beeinflussung an die Bohrerspitze weitergeleitet, so dass sich letztendlich der Widerstandswert des zwischen der Bohrerspitze und der gegenüberliegenden Gegenelektrode vorhandenen Gewebes präzise messen lässt. Auch der Bereich des Bohrerschafts, der in dem Bohrfutter eingespannt ist, sollte elektrisch isoliert sein, um eine Beeinflussung der Widerstandsmesswerte, z. B. durch das Bohrfutter oder den dahinterliegenden Bohrantrieb, zu vermeiden.

Weiterhin ist eine Bohrtiefenmessvorrichtung vorgesehen, mit der der Vorschub des Bohrwerkzeugs gemessen wird. Sofern diese Messung zu einem Zeitpunkt beginnt, an dem der Bohrer gerade mit dem Bohrvorgang beginnt, d. h. in den Knochen eindringt, lässt sich auf diese Weise die Tiefe der erzeugten Bohrung präzise messen. Vorteilhafterweise kann der gemessene Vorschub und damit die Bohrungstiefe auf einer Anzeige dargestellt werden, um dem Operateur und seiner Assistenz wertvolle Informationen über den Bohrfortschritt zu vermitteln.

Die Bohrtiefenmessvorrichtung kann ein mechanisches, federmechanisches, magnetisches, elektromagnetisches, induktives, kapazitives oder optisches Messprinzip aufweisen.

Der Zeitpunkt des Bohrbeginns, d. h. des Eindringens des Bohrwerkzeugs in den Knochen, kann von dem Bediener in vorteilhafter Weise mit Hilfe eines Signalgebers an die Bohrtiefenmessvorrichtung übertragen werden, um dadurch die Tiefe der nachfolgend entstehenden Bohrung präzise zu bestimmen.

Sämtliche Materialien und Gegenstände der Vorrichtung müssen vorzugsweise einer Sterilisierung in Wasserdampf bei einer Temperatur von 130 °C standhalten.

Die erfindungsgemäße Bohrvorrichtung und das mit Hilfe dieser Vorrichtung durchführbare Bohrverfahren ermöglichen es dem Operateur, ohne Unterbrechung des Bohrvorgangs Informationen über die Bohrungstiefe und über den Fortgang des Bohrvorgangs zu erhalten. Dadurch lässt sich einerseits eine deutliche Verkürzung der bisher erforderlichen Arbeitszeit für das Erzeugen von chirurgischen Bohrungen erreichen. Andererseits können Fehlbohrungen und daraus resultierende Schädigungen des Gewebes vermieden werden. Schließlich lässt sich mit Hilfe der präzise gemessenen Bohrungstiefe auch die Länge der darin einzupassenden Schrauben mit hoher Genauigkeit bestimmen. Intraoperative Röntgenkontrollen können dadurch auf ein Minimum reduziert werden.

Diese und weitere Vorteile und Merkmale der Erfindung werden nachfolgend anhand von Ausführungsbeispielen unter Zuhilfenahme der begleitenden Figuren näher erläutert. Es zeigen
- **Fig. 1**: eine schematische Skizze zum Erläutern einer ersten Ausführungsform der erfindungsgemäßen Bohrvorrichtung;
- **Fig. 2**: eine schematische Darstellung einer zweiten Ausführungsform der Erfindung;
- **Fig. 3**: eine schematische Darstellung einer dritten Ausführungsform der Erfindung; und
- **Fig. 4**: eine schematische Darstellung einer vierten Ausführungsform der Erfindung.

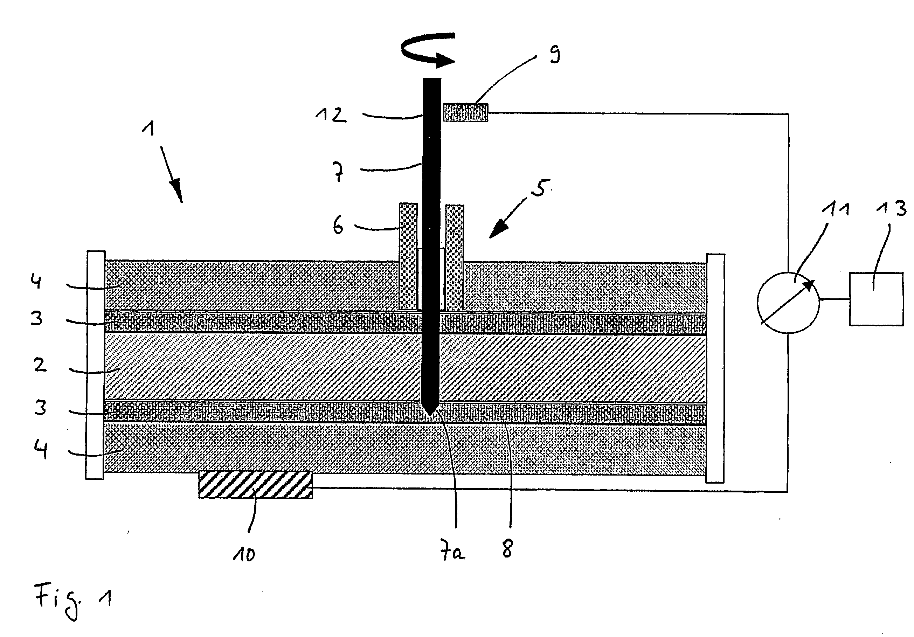

Fig. 1 zeigt in schematischer Form den prinzipiellen Aufbau einer erfindungsgemäßen Bohrvorrichtung während eines Bohrvorgangs beim medizinischen Bohren eines Knochens 1. Der Knochen 1 in Form eines Röhrenknochens ist im Schnitt dargestellt. In seinem Kern befindet sich Knochenmark 2 (Spongiosa), die im Wesentlichen zylindrisch von Knochenrinde 3 (Corticalis) umgeben ist. Die härtere Knochenrinde 3 wiederum kann von Weichgewebe 4, wie z. B. Muskeln oder Fett umgeben sein.

Das Weichgewebe 4 ist an einer Bohrstelle 5 chirurgisch entfernt, so dass die Knochenrinde 3 freigelegt ist. An dieser Stelle ist eine Gewebeschutzhülse 6 in bekannter Weise eingesetzt. Die Gewebeschutzhülse 6 dient einerseits dazu, eine Beschädigung des Weichgewebes 4 an der Bohrstelle 5 zu vermeiden. Weiterhin umschließt sie mit Spiel einen als Bohrwerkzeug dienenden Bohrer 7 und dient somit zu dessen Führung und Fixierung. Insbesondere beim Anbohren der Knochenrinde 3 kann dadurch vermieden werden, dass eine Spitze 7a des Bohrers 7 abrutscht und das Weichgewebe 4 schädigt.

Wie in Fig. 1 erkennbar, hat die Spitze 7a des Bohrers 7 zunächst die Knochenrinde 3 und dann das weichere Knochenmark 2 durchdrungen und befindet sich jetzt wieder in der Knochenrinde 3. Zur besseren Kennzeichnung wird dieser Teil der Knochenrinde 3 als Gegencorticalis 8 gekennzeichnet. Im weiteren Verlauf des Bohrvorgangs wird der Bohrer 7 die Gegencorticalis 8 durchbohren und in das dahinterliegende Weichgewebe 4 einbringen. Zur Vermeidung von Schädigungen des Weichgewebes 4 muss der Operateur besonders sorgfältig auf den Zeitpunkt achten, an dem die Gegencorticalis 8 durchbohrt wird.

Bei der erfindungsgemäßen Bohrvorrichtung wird der Bohrer 7 durch eine Bohrelektrode 9 kontaktiert. Gegenüber von dem Bohrer 7 bzw. der Bohrerspitze 7a ist außen an dem Weichgewebe 4, vorzugsweise an einer leicht zugänglichen Stelle, eine Gegenelektrode 10 angeordnet.

Die Bohrelektrode 9 und die Gegenelektrode 10 sind mit einer Widerstandsmesseinrichtung 11 verbunden, die die Elektroden 9, 10 mit einer Gleich-oder Wechselspannung beaufschlagt und den elektrischen Widerstand zwischen den beiden Elektroden 9, 10 misst.

Der Bohrer 7 ist ein üblicher Bohrer aus einem elektrisch leitenden Metall. Jedoch ist seine Außenhaut bis auf die Bohrerspitze 7a und eine Ringfläche 12 am Bohrerschaft elektrisch isoliert, z. B. durch Galvanisierung oder Eloxierung.

Die Bohrelektrode 9 kontaktiert die elektrisch leitende Ringfläche 12. Dadurch wird die Gleich- oder Wechselspannung von der Bohrelektrode 9 auf die Ringfläche 12 und damit direkt auf die Bohrerspitze 7a übertragen.

Prinzipiell ist auf diese Weise die Messung eines Gleichspannungswiderstands des Gewebes zwischen der Bohrerspitze 7a und der Gegenelektrode 10 möglich. Es hat sich jedoch herausgestellt, dass eine Beaufschlagung mit einer Wechselspannung und eine Messung der Impedanz besonders präzise Ergebnisse liefert.

Die Impedanzmessung erfolgt aus verständlichen Gründen mit möglichst niedrigen Spannungen und Strömen, so z. B. 1 Volt. Die Wechselspannung wird durch die Widerstandsmesseinrichtung 11 aufgeprägt und die Impedanz aus dem gemessenen Strom berechnet. Hierzu eignet sich besonders die sogenannte Lock-In-Technik, die an sich bekannt ist, so dass sich eine weitergehende Beschreibung erübrigt. Die Frequenz der Wechselspannung liegt typischerweise zwischen 1 kHz und 100 kHz, wodurch sich Störströme aufgrund des 50 Hz Brummens oder aufgrund von hochfrequenten Einkopplungen vermeiden lassen. Vielmehr wird der Strom nur bei der vorgegebenen Frequenz detektiert (Lock-In), so dass die Störströme anderer Frequenzen unberücksichtigt bleiben. Neben der Amplitude der Impedanz wird auch deren Phase bestimmt. Unter Verwendung geeigneter Bandpassfilter wird das gemessene Signal weiter aufbereitet.

Da es für den Operateur - wie oben bereits dargelegt - besonders wichtig ist, den Zeitpunkt des Durchbohrens der Gegencorticalis 8 zu erkennen, können geeignete Maßnahmen zur Detektion des aus der Gewebeänderung resultierenden Impedanzsprungs ergriffen werden. So lässt sich z. B. dieser Sprung durch geeignete Filter, z. B. durch Differenzieren des Signals und Bestimmung des Maximums erkennen.

Die Widerstandsmesseinrichtung 11 ist mit einer Anzeigeeinrichtung 13 verbunden, die zum Auswerten der von der Widerstandsmesseinrichtung erfassten Messwerte dient. Dazu ist vorteilhafterweise in der Anzeigeeinrichtung 13 ein Computer vorgesehen, der die Messwerte speichert, aufbereitet und in geeigneter Weise darstellt. So kann z. B. auf einem Display der Gewebetyp (Knochenrinde 3, Knochenmark 2, Weichgewebe 4, Gegencorticalis 8) dargestellt werden, in dem sich die Bohrerspitze 7a gerade befindet. Beim Durchdringen der Gegencorticalis 8 und Eindringen in das dahinterliegende Weichgewebe 4 kann sowohl ein optisches als auch ein akustisches Signal erzeugt werden. Weiterhin kann die Anzeigeeinrichtung 13 zum Erfassen und Ausgeben der Bohrungstiefe genutzt werden, was später noch erläutert wird.

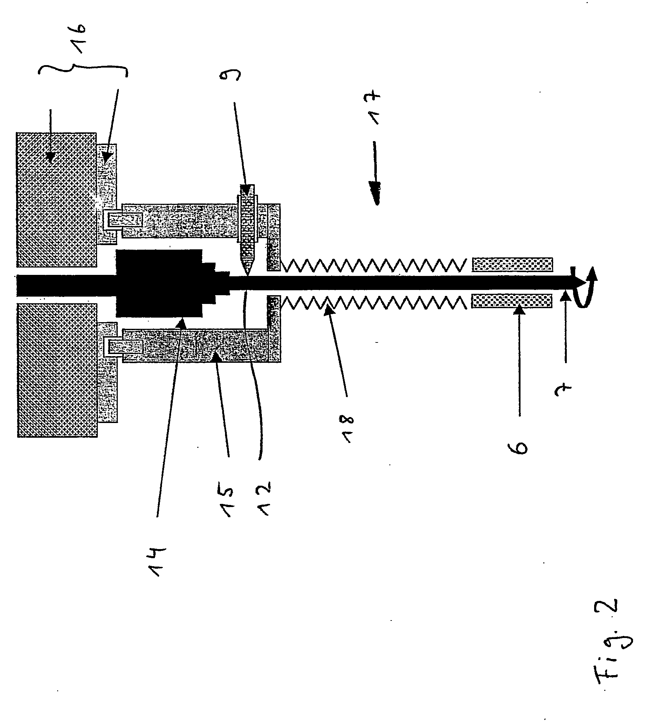

Fig. 2 zeigt eine zweite Ausführungsform der erfindungsgemäßen Bohrvorrichtung, wobei die oben beschriebene Widerstandsmessung unverändert beibehalten wird.

Als Detaillierung zu dem in Fig. 1 erläuterten Schema ist in Fig. 2 darüber hinaus zu erkennen, dass der Bohrer 7 in einem Bohrfutter 14 eingespannt ist. Das Bohrfutter 14 wird in bekannter Weise durch einen nicht dargestellten Bohrantrieb drehend angetrieben.

Die Bohrelektrode 9 ist in Form eines an sich bekannten Kohleschleifers zur elektrischen Kontaktierung der Ringfläche 12 des Bohrers 7 ausgebildet. Der Kohleschleifer wird über eine nicht dargestellte Feder gegen die Ringfläche 12 gedrückt. Selbstverständlich sind zur Kontaktierung der Bohrelektrode 9 mit der Ringfläche 12 auch zahlreiche andere Möglichkeiten bekannt, die an dieser Stelle nicht vertieft werden müssen.

Zur besseren Führung der Bohrelektrode 9 ist diese in einen Gehäuseaufsatz 15 integriert, die wiederum in geeigneter Weise, z. B. mit Hilfe eines Bajonettverschlusses an einem auch den Bohrantrieb aufnehmenden Hauptgehäuse 16 der Bohrvorrichtung befestigt ist.

Weiterhin ist bei der zweiten Ausführungsform der Erfindung eine Bohrtiefenmessvorrichtung 17 in Ergänzung zu der oben beschriebenen Widerstandsmesseinrichtung vorgesehen.

Die Bohrtiefenmessvorrichtung 17 ist in Fig. 2 nur schematisch dargestellt und kann folgenden Aufbau aufweisen: Zwischen dem Hauptgehäuse 16 oder dem Gehäuseaufsatz 15 der Bohrvorrichtung und der Gewebeschutzhülse 6 ist ein dehn - und stauchbares mechanisches Element, z. B. eine Feder 18 eingesetzt. Die Feder kann z. B. lediglich auf einer Seite, z. B. an dem Gehäuseaufsatz 15 befestigt sein, während sie auf der Gewebeschutzhülse 6 lose aufliegt. Beim Vorschub des Bohrers 7 wird die Feder 18 gestaucht, während sie sich beim Zurückziehen des Bohrers 7 entspannen kann. Die in der Feder 18 wirkende Federkraft kann mit Hilfe einer nicht dargestellten, an der Federhalterung vorgesehenen Kraftmessdose oder einem Dehnmessstreifen gemessen werden. In Kenntnis der Federkennlinie der Feder 18 lässt sich daraus der Stauchungsweg der Feder 18 und somit der Vorschub des Bohrers 7 relativ zu der Gewebeschutzhülse 6 errechnen und z. B. über die Anzeigeeinrichtung 13 (Fig. 1) ausgeben.

Über eine nicht dargestellt Signaleinrichtung hat der Operateur die Möglichkeit, den Beginn der Bohrtiefenmessung zu bestimmen. Dazu kann er z. B. den Signalgeber genau in dem Moment betätigen, in dem die Bohrerspitze 7a beginnt, in den Knochen einzudringen. Ab diesem Zeitpunkt wird der Bohrvorschub gemessen, wobei ein gelegentliches Abheben des Bohrers 7 und damit eine Unterbrechung des Bohrvorgangs unschädlich ist, weil die in der Anzeigeeinrichtung 13 vorgesehene Auswerteeinrichtung die jeweiligen Vorschubänderungen in beiden Richtungen erfasst und derart addiert, dass stets der Absolutvorschub gegenüber der Ausgangslage, in der die Messung begonnen wurde, ermittelt wird.

In Kombination mit der oben beschriebenen Widerstandsmesseinrichtung erhält somit der Operateur über die Anzeigeeinrichtung 13 ständige Informationen über die Bohrungstiefe sowie über den zum jeweiligen Zeitpunkt gebohrten Gewebetyp bzw. eine Änderung des Gewebetyps bei Durchbohren der jeweiligen Gewebegrenzen.

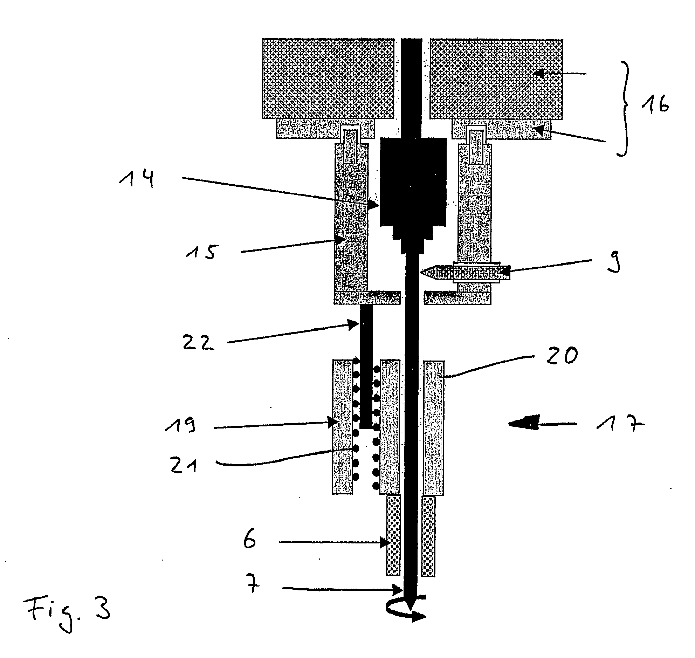

Fig. 3 zeigt schematisch eine dritte Ausführungsform der Erfindung, die im Wesentlichen auf der bereits erläuterten zweiten Ausführungsform der Erfindung aufbaut. Im Unterschied zu der zweiten Ausführungsform gemäß Fig. 2 basiert jedoch die Bohrtiefenmessvorrichtung 17 auf einem magnetischen bzw. elektromagnetischen Prinzip.

Zu diesem Zweck ist vorzugsweise an der Gewebeschutzhülse 6 ein Aufsatz 19 in die Gewebeschutzhülse 6 integriert oder separat davon vorgesehen, der einerseits einen Durchbruch 20 zur Durchführung des Bohrers 7 und andererseits eine Spule 21 aufweist. Die Spule 21 ist vorzugsweise vollständig gekapselt, um ihre Reinigung und Sterilisierung zu erleichtern.

An dem Hauptgehäuse 16 oder dem Gehäuseaufsatz 15 der Bohrvorrichtung ist ein Ferritstab 22 befestigt, der ebenfalls gekapselt sein sollte. Der Ferritstab 22 dringt in die Spule 21 ein und dient somit als Spulenkern. Je nach Eindringtiefe des Ferritstabes 22 ändert sich die Induktivität der Spule 21, woraus der Vorschub des Bohrers 7 errechnet und auf der Anzeigeeinrichtung 13 angezeigt werden kann.

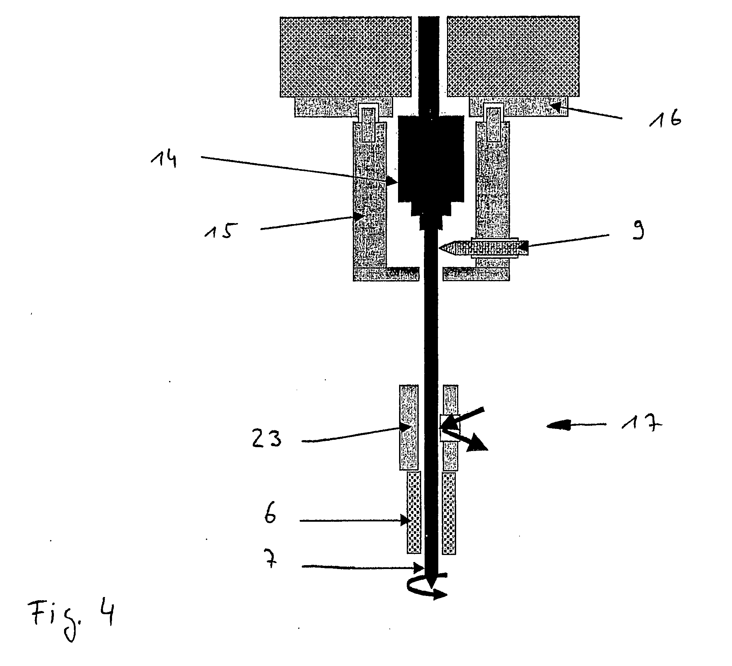

Fig. 4 zeigt schließlich eine vierte Ausführungsform der Erfindung, die sich von den in den Fig. 2 und 3 gezeigten Ausführungsformen in dem Prinzip der Bohrtiefenmessvorrichtung 17 unterscheidet. Anstelle der bisher erläuterten mechanischen und magnetischen Bohrtiefenmessung wird bei der vierten Ausführungsform ein optisches Messverfahren verwendet.

Dazu werden auf dem Bohrer 7 schmale farbige Ringe, z. B. im Abstand von 0,5 mm als Markierung aufgebracht. Die Farben können sich z. B. in folgender Weise ändern: Weiß, Rot, Grün, Weiß, Rot, Grün, Weiß, ....

Auf die Gewebeschutzhülse 6 ist eine Messhülse 23 aufgesetzt. Die Messhülse 23 kann aber auch in die Gewebeschutzhülse 6 integriert sein. In der Messhülse 23 sind zwei nicht dargestellte, auf den Bohrer 7 fokussierte Lichtquellen vorgesehen, wobei das Licht von der ersten Lichtquelle z. B. sowohl von der grünen als auch von der weißen Schicht reflektiert wird, während das Licht von der zweiten Lichtquelle sowohl von der roten als auch von der weißen Schicht reflektiert wird. Das reflektierte Licht von jeder Lichtquelle wird von einer nicht dargestellten Fotodiode erfasst. In einer ebenfalls nicht dargestellten Auswerteeinheit werden die von den Lichtquellen detektierten Farbänderungen erfasst, gezählt und hinsichtlich der Farbänderungen registriert. Durch das Abzählen der Ringe lässt sich sowohl die Richtung als auch der Bohrvorschub berechnen. Zum Beispiel kann das System auf ein Bohren in Vorschubrichtung schießen, wenn die Farbe von Weiß nach Grün wechselt, während ein Rückziehen des Bohrers registriert wird, wenn die Farbe von Weiß nach Rot wechselt. Pro gezähltem Ring wird ein Vorschub von z. B. 0,5 mm ermittelt, so dass die Auflösung dieses Systems ebenfalls 0,5 mm beträgt. Ein an der Oberseite der Gewebeschutzhülse 6 oder der Messhülse 23 vorgesehener nicht dargestellter Blutabstreifer verhindert die Verschmutzung der Ringe mit Blut oder Knochenmehl.

Die in den Fig. 2 bis 4 gezeigten Ausführungsbeispiele dienen lediglich zur Erläuterung von prinzipiellen Lösungen für eine Bohrtiefenmessvorrichtung. Selbstverständlich sind aus anderen technischen Gebieten, z. B. der Bohrtechnik oder dem Werkzeugbau, weitere Lösungen bekannt, mit denen die Tiefe einer Bohrung ermittelt werden kann. Hierbei ist jedoch zu beachten, dass die Bohrvorrichtung durch den Operateur vorteilhafterweise von Hand geführt wird, weil der Operateur ein "Gefühl" für den Bohrvorgang haben muss. Die Bohrtiefenmessvorrichtung muss daher auch bei einer Handführung des Bohrgeräts und der damit verbundenen Änderungen der Winkelstellungen und einem gelegentlichen Absetzen des Bohrers arbeiten können. Die Bohrvorrichtung kann selbstverständlich auch in einer Halterung eingespannt oder gar von einem Roboter geführt werden.

Zur Verfeinerung der Messgenauigkeit kann zusätzlich zu der Messung der Impedanz, insbesondere des Impedanzsprungs, der auftritt, wenn die Bohrerspitze von einem Gewebetyp in einen anderen Gewebetyp eindringt, auch das Drehmoment des Bohrerantriebs gemessen werden. Je nach durchbohrtem Gewebe wird der Bohrer mit einem bestimmten Bremsmoment abgebremst, das von dem Bohrerantrieb überwunden werden muss. Zwar wird das Bremsmoment auch durch die Reibung an den zylindrischen Seitenwänden des Bohrers beeinflusst. Jedoch hat sich herausgestellt, dass das Bremsmoment bzw. Bohrmoment im Wesentlichen durch die Schneidkräfte an der Bohrerspitze hervorgerufen wird.

Die Messung des Drehmoments kann auf verschiedene Weise erfolgen. Z. B. ist es möglich, die Stromaufnahme des Bohrerantriebs zu messen und dadurch Rückschlüsse auf das Drehmoment zu ziehen.

Die Information über das Drehmoment des Bohrers kann zusammen mit der Impedanzinformation, insbesondere beim Feststellen eines Impedanzsprungs, mit Hilfe der Auswerteeinheit ausgewertet werden, um dem Operateur eine präzise Information darüber zu liefern, welches Gewebe gerade gebohrt wird, insbesondere ob die Bohrerspitze zu einem bestimmten Zeitpunkt von einem Gewebetyp in einen anderen Gewebetyp eindringt.

In dem Moment, wenn z. B. die Bohrerspitze aus der Spongiosa in die Gegencorticalis eindringt, lässt sich nicht nur ein Impedanzsprung detektieren, sondern auch ein erhöhtes Bremsmoment am Bohrer messen. Die Auswerteeinheit kann aufgrund dieser beiden Änderungen, die z. B. innerhalb eines gewissen, durch Experimente im Vorfeld feststellbaren Zeitraums auftreten müssen, dem Operateur zuverlässige Informationen zur Verfügung stellen.

Da das am Bohrer auftretende Drehmoment von den Schneidkräften abhängt und diese wiederum abhängig sind von der Andrückkraft, mit der der Operateur den Bohrer gegen den Knochen drückt, kann es darüber hinaus zweckmäßig sein, wenn auch die Andrückkraft gemessen wird. Dazu eignet sich z. B. eine entsprechende Kraftmesseinrichtung in dem Bohrer-Handstück.

Die Information über die Andrückkraft kann dazu dienen, das jeweils konkret gemessene Drehmoment in einen normierten bzw. standardisierten Drehmomentwert umzurechnen. Auch hierzu liegen der Auswerteeinheit entsprechende Möglichkeiten vor, um eine weitere Erhöhung der Messgenauigkeit zu erreichen.

## Patentansprüche

1. Medizinische Bohrvorrichtung zum Bohren von menschlichem oder tierischem Gewebe (2, 3, 4), mit
- einem Bohrantrieb zum drehenden Antreiben eines elektrisch leitenden Bohrwerkzeugs (7), **dadurch gekennzeichnet, dass** die Bohrvorrichtung weiterhin ausgestattet ist, mit
- einer Bohrelektrode (9), die mit dem Bohrwerkzeug (7) in elektrischem Kontakt steht,
- einer Gegenelektrode (10), die derart anordenbar ist, dass sich das zu bohrende Gewebe im Wesentlichen zwischen der Gegenelektrode (10) und dem Bohrwerkzeug (7) befindet,
- einer Widerstandsmesseinrichtung (11), die mit der Bohrelektrode (9) und der Gegenelektrode (10) verbunden und derart ausgebildet ist, dass die beiden Elektroden (9, 10) mit einem Strom beaufschlagbar sind und der elektrische Widerstand des zwischen dem von der Bohrelektrode (9) kontaktierten Bohrwerkzeug (7) und der Gegenelektrode (10) befindlichen Gewebes (2, 3, 4) messbar ist, und mit
- einer Anzeigeeinrichtung (13) zum Anzeigen von Information aufgrund von von der Widerstandsmesseinrichtung (11) erfassten Messwerten; wobei
- das Bohrwerkzeug ein Bohrer (7) aus einem elektrisch leitenden Material ist, der eine isolierende Ummantelung derart aufweist, dass die Bohrerspitze (7a) sowie ein ringförmiger Bereich (12) am Bohrerschaft frei von der Ummantelung und somit zur Umgebung hin elektrisch leitend sind.

2. Medizinische Bohrvorrichtung nach Anspruch 1,
**dadurch gekennzeichnet, dass**
- eine Bohrtiefenmessvorrichtung (17) vorgesehen ist, zum Messen des Vorschubs des Bohrwerkzeugs (7) ab einem definierbaren Zeitpunkt oder einer definierbaren Ausgangsposition.

3. Medizinische Bohrvorrichtung zum Bohren von menschlichem oder tierischem Gewebe (2, 3, 4), mit
- einem Bohrantrieb zum drehenden Antreiben eines elektrisch leitenden Bohrwerkzeugs (7), **dadurch gekennzeichnet, dass** die Bohrvorrichtung weiterhin ausgestattet ist, mit
- einer Bohrelektrode (9), die mit dem Bohrwerkzeug (7) in elektrischem Kontakt steht,
- einer Gegenelektrode (10), die derart anordenbar ist, dass sich das zu bohrende Gewebe im Wesentlichen zwischen der Gegenelektrode (10) und dem Bohrwerkzeug (7) befindet,
- einer Widerstandsmesseinrichtung (11), die mit der Bohrelektrode (9) und der Gegenelektrode (10) verbunden und derart ausgebildet ist, dass die beiden Elektroden (9, 10) mit einem Strom beaufschlagbar sind und der elektrische Widerstand des zwischen dem von der Bohrelektrode (9) kontaktierten Bohrwerkzeug (7) und der Gegenelektrode (10) befindlichen Gewebes (2, 3, 4) messbar ist, und mit
- einer Anzeigeeinrichtung (13) zum Anzeigen von Information aufgrund von von der Widerstandsmesseinrichtung (11) erfassten Messwerten; wobei
- eine Bohrtiefenmessvorrichtung (17) vorgesehen ist, zum Messen des Vorschubs des Bohrwerkzeugs (7) ab einem definierbaren Zeitpunkt oder einer definierbaren Ausgangsposition.

4. Medizinische Bohrvorrichtung nach einem der Ansprüche 1 bis 3, **dadurch gekennzeichnet, dass**
- eine Drehmomenterfassungseinrichtung vorgesehen ist, zum Erfassen des an dem Bohrwerkzeug (7) während eines Bohrvorgangs angreifenden Drehmoments.

5. Medizinische Bohrvorrichtung zum Bohren von menschlichem oder tierischem Gewebe (2, 3, 4), mit
- einem Bohrantrieb zum drehenden Antreiben eines elektrisch leitenden Bohrwerkzeugs (7), **dadurch gekennzeichnet, dass** die Bohrvorrichtung weiterhin ausgestattet ist, mit
- einer Bohrelektrode (9), die mit dem Bohrwerkzeug (7) in elektrischem Kontakt steht,
- einer Gegenelektrode (10), die derart anordenbar ist, dass sich das zu bohrende Gewebe im Wesentlichen zwischen der Gegenelektrode (10) und dem Bohrwerkzeug (7) befindet,
- einer Widerstandsmesseinrichtung (11), die mit der Bohrelektrode (9) und der Gegenelektrode (10) verbunden und derart ausgebildet ist, dass die beiden Elektroden (9, 10) mit einem Strom beaufschlagbar sind und der elektrische Widerstand des zwischen dem von der Bohrelektrode (9) kontaktierten Bohrwerkzeug (7) und der Gegenelektrode (10) befindlichen Gewebes (2, 3, 4) messbar ist, und mit
- einer Anzeigeeinrichtung (13) zum Anzeigen von Information aufgrund von von der Widerstandsmesseinrichtung (11) erfassten Messwerten; wobei
- eine Drehmomenterfassungseinrichtung vorgesehen ist, zum Erfassen des an dem Bohrwerkzeug (7) während eines Bohrvorgangs angreifenden Drehmoments.

6. Medizinische Bohrvorrichtung nach einem der Ansprüche 1 bis 5, **dadurch gekennzeichnet, dass** die Widerstandsmesseinrichtung (11) eine Impedanzmesseinrichtung ist, dass der Strom ein Wechselstrom ist und dass der elektrische Widerstand eine elektrische Impedanz ist.

7. Medizinische Bohrvorrichtung nach einem der Ansprüche 1 bis 6, **dadurch gekennzeichnet, dass** die Anzeigeeinrichtung (13) zum Anzeigen von optischen oder akustischen Signalen aufgrund von aus der Widerstandsmesseinrichtung (11) gewonnenen Messwerten oder Messwertänderungen ausgebildet ist.

8. Medizinische Bohrvorrichtung nach einem der Ansprüche 1 bis 7, **dadurch gekennzeichnet, dass** die Anzeigeeinrichtung (13) eine Auswerteeinheit aufweist, mit der aufgrund der von der Widerstandsmesseinrichtung (11) erfassten Messwerte oder Messwertänderungen Information über die Qualität oder den Typ des Gewebes ermittelbar ist, welches zu dem jeweiligen Zeitpunkt durch das Bohrwerkzeug (7) gebohrt wird.

9. Medizinische Bohrvorrichtung nach Anspruch 8, **dadurch gekennzeichnet, dass** durch die Auswerteeinheit eine Schalteinrichtung ansteuerbar ist, um den Bohrantrieb abzuschalten oder um über die Anzeigeeinrichtung (13) ein entsprechendes optisches oder akustisches Signal abzugeben, wenn die Auswerteeinheit das Bohren eines bestimmten Gewebetyps feststellt.

10. Medizinische Bohrvorrichtung nach einem der Ansprüche 1 bis 9, **dadurch gekennzeichnet, dass** die Bohrelektrode (9) den Bohrer (7) an dem ringförmigen Bereich (12) elektrisch kontaktiert.

11. Medizinische Bohrvorrichtung nach einem der Ansprüche 2 bis 10, **dadurch gekennzeichnet, dass** die Bohrtiefenmessvorrichtung (17) mit einem Signalgeber gekoppelt ist, mit dem von dem Bediener der Bohrvorrichtung der Zeitpunkt oder die Ausgangsposition zu Beginn des Messvorgangs an die Bohrtiefenmessvorrichtung (17) signalisierbar ist.

12. Medizinische Bohrvorrichtung nach einem der Ansprüche 2 bis 11, **dadurch gekennzeichnet, dass** durch die Bohrtiefenmessvorrichtung (17) der Vorschub des Bohrwerkzeugs (7) relativ zu einer Gewebeschutzhülse (6) messbar ist, die an dem Ort anordenbar ist, an dem das Bohrwerkzeug (7) in das zu bohrende Gewebe (2, 3, 4) eindringt, und die das Bohrwerkzeug (7) an diesem Ort führt.

13. Medizinische Bohrvorrichtung nach einem der Ansprüche 4 bis 12, **dadurch gekennzeichnet, dass**
- eine Andrückkraft-Messeinrichtung vorgesehen ist, zum Erfassen der Andrückkraft, mit der ein Operateur das Bohrwerkzeug (7) gegen das zu bohrende Gewebe andrückt, und dass
- eine Drehmoment-Auswerteeinrichtung vorgesehen ist, mit der die von der Drehmomenterfassungseinrichtung gemessenen Drehmomentwerte entsprechend der gemessenen Andrückkraftwerte normierbar sind.

14. Medizinische Bohrvorrichtung nach einem der Ansprüche 4 bis 13, **dadurch gekennzeichnet, dass** eine Drehmoment-Auswerteeinrichtung vorgesehen ist, mit der aufgrund der von der Drehmomenterfassungseinrichtung erfassten Messwerte oder Messwertänderungen und/oder der Andrückkraftwerte Information über die Qualität oder den Typ des Gewebes ermittelbar ist, welches zu dem jeweiligen Zeitpunkt durch das Bohrwerkzeug (7) gebohrt wird.

15. Medizinische Bohrvorrichtung nach Anspruch 8 und einem der Ansprüche 4 bis 14, **dadurch gekennzeichnet, dass** mit der Auswerteeinheit durch eine kombinierte Auswertung der von der Widerstandsmesseinrichtung (11) erfassten Messwerte oder Messwertänderungen und der von der Drehmomenterfassungseinrichtung erfassten Messwerte oder Messwertänderungen die Information über die Qualität oder den Typ des Gewebes ermittelbar ist, welches zu dem jeweiligen Zeitpunkt durch das Bohrwerkzeug (7) gebohrt wird.

## Claims

1. Medical drilling tool for drilling through human or animal tissue (2, 3, 4), comprising:
- a drilling mechanism for the rotary driving of an electrically conductive drilling tool (7), **characterised in that** the drilling tool is further equipped with:
- a drilling electrode (9), which is in electrical contact with the drilling tool (7),
- a counter-electrode (10), which can be arranged in such a way that effectively the tissue to be drilled lies between the counter-electrode (10) and the drilling tool (7),
- a resistance measuring device (11), which is connected to the drilling electrode (9) and the counter-electrode (10) and which is configured in such a way that the two electrodes (9, 10) can be exposed to a current and the electrical resistance of the tissue (2, 3, 4) positioned between the drilling tool (7), which is in contact with the drilling electrode (9), and the counter-electrode (10), can be measured and with
- a display device (13) to display information based on the values measured by the resistance measuring device (11),
whereby
- the drilling tool is a drill (7) in an electrically conductive material, which comprises an insulating cover in such a way that the tip of the drill (7a) and also a ring-shaped area (12) on the shaft of the drill are not affected by the said cover and are thus electrically conductive in respect of the surrounding area.

2. Medical drilling tool according to Claim 1 above,
**characterised in that**
- a drilling depth measuring device (17) is provided in order to measure the forward movement of the drilling tool (7) with effect from a pre-definable point in time or a definable starting position.

3. Medical drilling tool for drilling human or animal tissue (2, 3, 4), comprising
- a drilling mechanism for the rotary driving of an electrically conductive drilling tool (7), **characterised in that** the drilling tool is further equipped with:
- a drilling electrode (9), which is in electrical contact with the drilling tool (7),
- a counter-electrode (10), which can be arranged in such a way that effectively the tissue to be drilled lies between the counter-electrode (10) and the drilling tool (7),
- a resistance measuring device (11), which is connected to the drilling electrode (9) and the counter-electrode (10) and which is configured in such a way that the two electrodes (9, 10) can be exposed to a current and the electrical resistance of the tissue (2, 3, 4) positioned between the drilling tool (7), which is in contact with the drilling electrode (9), and the counter-electrode (10), can be measured and with
- a display device (13) to display information based on the values measured by the resistance measuring device (11),
whereby
- a drilling depth measuring device (17) is provided for the purpose of measuring the forward movement of the drilling tool (7) from a definable point in time or from a definable starting position.

4. Medical drilling tool according to any of Claims 1 to 3 above, **characterised in that**
- a torque calculation device is provided for the purpose of determining the torque to which the drilling tool (7) is subjected during a drilling operation.

5. Medical drilling tool for the drilling of human or animal tissue (2, 3, 4), comprising:
- a drilling mechanism for the rotary driving of an electrically conductive drilling tool (7), **characterised in that** the drilling tool is further equipped with:
- a drilling electrode (9), which is in electrical contact with the drilling tool (7),
- a counter-electrode (10), which can be arranged in such a way that effectively the tissue to be drilled lies between the counter-electrode (10) and the drilling tool (7),
- a resistance measuring device (11), which is connected to the drilling electrode (9) and the counter-electrode (10) and which is configured in such a way that the two electrodes (9, 10) can be exposed to a current and the electrical resistance of the tissue (2, 3, 4) positioned between the drilling tool (7), which is in contact with the drilling electrode (9), and the counter-electrode (10), can be measured and with:
- a display device (13) to display information based on the values measured by the resistance measuring device (11),
whereby
- a torque measuring device is provided for the purpose of determining the torque to which the drilling tool (7) is subjected during a drilling operation.

6. Medical drilling tool according to any of the Claims 1 to 5 above, **characterised in that** the resistance measuring device (11) is an impedance measuring device, that the current is an alternating current and that the electrical resistance is an impedance.

7. Medical drilling tool according to any of the Claims 1 to 6 above, **characterised in that** the display device (13) is designed in such a way that it displays optical or acoustic signals on the basis of measurements or amended measurements obtained by the resistance measuring device (11).

8. Medical drilling tool according to any of the Claims 1 to 7 above, **characterised in that** the display device (13) comprises an evaluation device, by means of which on the basis of the measurements or amended measurements obtained by the resistance measuring device (11), information can be obtained on the quality or the type of the tissue that is being drilled at a given moment by the drilling tool (7).

9. Medical drilling tool according to Claim 8 above, **characterised in that**, through the evaluation unit, a switching device can be controlled so that the drilling operation can be stopped or so that, through the display device (13), a corresponding optical or acoustic signal can be emitted if the evaluation device detects that a certain type of tissue is being drilled.

10. Medical drilling tool according to any of Claims 1 to 9 above, **characterised in that** the drilling electrode (9) makes electrical contact with the drilling tool (7) in the circular shaped area (12).

11. Medical drilling tool according to any of Claims 2 to 10 above, **characterised in that** the drilling depth measuring device (17) is coupled to a signal emitter, with which signals can be sent by the operator of the drilling tool to the drilling depth measuring device (17) on the time or the starting position at the start of the measuring process.

12. Medical drilling tool according to any of Claims 2 to 11 above, **characterised in that**, by means of the drilling depth measuring device (17), the forward movement of the drilling tool (7) in relation to a protective sleeve for the tissue (6) can be measured, and the protective sleeve can be positioned at a place at which the drilling tool (7) penetrates the tissue (2, 3, 4) to be drilled and guides the drilling tool at this place.

13. Medical drilling tool according to any of Claims 4 to 12 above, **characterised in that**:
- a device for measuring the applied pressure is provided for the purpose of measuring the pressing force with which an operator presses the drilling tool (7) against the tissue to be drilled, and that
- a torque evaluation device is provided, with which the torque values measured by the torque determination device can be scaled against the measured pressing force values.

14. Medical drilling tool according to any of Claims 4 to 13 above, **characterised in that** a torque evaluation device is provided, by means of which, on the basis of the measurements or amended measurements and/or the pressing force values obtained from the torque determination device, information can be obtained on the quality or the type of the tissue that is being drilled at a given point in time by the drilling tool (7).

15. Medical drilling tool according to Claim 8 above and any of Claims 4 to 14 above, **characterised in that** with the evaluation unit, by means of a combined evaluation of the measurements or amended measurements calculated by the resistance measuring device (11) and the measurements or amended measurements obtained from the torque determination device, information can be obtained on the quality or the type of the tissue that is being drilled at any particular time by the drilling tool (7).

## Revendications

1. Dispositif médical de perçage pour percer un tissu humain ou animal (2, 3, 4), avec
- un entraînement de perçage pour entraîner en rotation un outil de perçage (7) conducteur d'électricité, **caractérisé en ce que** le dispositif de perçage est également équipé
- d'une électrode de perçage (9) qui est en contact électrique avec l'outil de perçage (7),
- d'une contre-électrode (10) qui est apte à être disposée de telle sorte que le tissu à percer se trouve globalement entre la contre-électrode (10) et l'outil de perçage (7),
- d'un dispositif de mesure de résistance (11) qui est relié à l'électrode de perçage (9) et à la contre-électrode (10) et qui est conçu de telle sorte que les deux électrodes (9, 10) soient aptes à être sollicitées par un courant et que la résistance électrique du tissu (2, 3, 4) situé entre l'outil de perçage (7) avec lequel l'électrode de perçage (9) est en contact, et la contre-électrode (10) puisse être mesurée, et
- d'un dispositif d'affichage (13) pour afficher des informations sur la base de valeurs de mesure relevées par le dispositif de mesure de résistance (11) ;
étant précisé
- que l'outil de perçage est constitué par un foret (7) en matériau conducteur d'électricité, qui présente une gaine isolante de telle sorte que la pointe (7a) du foret et une zone annulaire (12) sur la tige du foret soient dégagées de la gaine et soient ainsi conductrices d'électricité en direction de l'environnement.

2. Dispositif médical de perçage selon la revendication 1, **caractérisé en ce**
- **qu'**il est prévu un dispositif de mesure de profondeur de perçage (17) pour mesurer l'avance de l'outil de perçage (7) à partir d'un moment apte à être défini ou d'une position de départ apte à être définie.

3. Dispositif médical de perçage pour percer un tissu humain ou animal (2, 3, 4), avec
- un entraînement de perçage pour entraîner en rotation un outil de perçage (7) conducteur d'électricité, **caractérisé en ce que** le dispositif de perçage est également équipé
- d'une électrode de perçage (9) qui est en contact électrique avec l'outil de perçage (7),
- d'une contre-électrode (10) qui est apte à être disposée de telle sorte que le tissu à percer se trouve globalement entre la contre-électrode (10) et l'outil de perçage (7),
- d'un dispositif de mesure de résistance (11) qui est relié à l'électrode de perçage (9) et à la contre-électrode (10) et qui est conçu de telle sorte que les deux électrodes (9, 10) soient aptes à être sollicitées par un courant et que la résistance électrique du tissu (2, 3, 4) situé entre l'outil de perçage (7) avec lequel l'électrode de perçage (9) est en contact, et la contre-électrode (10) puisse être mesurée, et
- d'un dispositif d'affichage (13) pour afficher des informations sur la base de valeurs de mesure relevées par le dispositif de mesure de résistance (11) ;
étant précisé
- qu'il est prévu un dispositif de mesure de profondeur de perçage (17) pour mesurer l'avance de l'outil de perçage (7) à partir d'un moment apte à être défini ou d'une position de départ apte à être définie.

4. Dispositif médical de perçage selon l'une des revendications 1 à 3, **caractérisé en ce**
- **qu'**il est prévu un dispositif de relevé de couple pour relever le couple qui agit sur l'outil de perçage (7) pendant une opération de perçage.

5. Dispositif médical de perçage pour percer un tissu humain ou animal (2, 3, 4), avec
- un entraînement de perçage pour entraîner en rotation un outil de perçage (7) conducteur d'électricité, **caractérisé en ce que** le dispositif de perçage est également équipé
- d'une électrode de perçage (9) qui est en contact électrique avec l'outil de perçage (7),
- d'une contre-électrode (10) qui est apte à être disposée de telle sorte que le tissu à percer se trouve globalement entre la contre-électrode (10) et l'outil de perçage (7),
- d'un dispositif de mesure de résistance (11) qui est relié à l'électrode de perçage (9) et à la contre-électrode (10) et qui est conçu de telle sorte que les deux électrodes (9, 10) soient aptes à être sollicitées par un courant et que la résistance électrique du tissu (2, 3, 4) situé entre l'outil de perçage (7) avec lequel l'électrode de perçage (9) est en contact, et la contre-électrode (10) puisse être mesurée, et
- d'un dispositif d'affichage (13) pour afficher des informations sur la base de valeurs de mesure relevées par le dispositif de mesure de résistance (11) ;
étant précisé
- qu'il est prévu un dispositif de relevé de couple pour relever le couple qui agit sur l'outil de perçage (7) pendant une opération de perçage.

6. Dispositif médical de perçage selon l'une des revendications 1 à 5, **caractérisé en ce que** le dispositif de mesure de résistance (11) est constitué par un dispositif de mesure d'impédance, **en ce que** le courant est un courant alternatif, et **en ce que** la résistance électrique est constituée par une impédance électrique.

7. Dispositif médical de perçage selon l'une des revendications 1 à 6, **caractérisé en ce que** le dispositif d'affichage (13) est conçu pour afficher des signaux optiques ou acoustiques sur la base de valeurs de mesure ou de variations de valeurs de mesure qui sont recueillies à partir du dispositif de mesure de résistance (11).

8. Dispositif médical de perçage selon l'une des revendications 1 à 7, **caractérisé en ce que** le dispositif
d'affichage (13) comporte une unité d'analyse avec laquelle peuvent être obtenues, sur la base des valeurs de mesure ou des variations de valeurs de mesure relevées par le dispositif de mesure de résistance (11), des informations sur la qualité ou le type du tissu qui est percé par l'outil de perçage (7) au moment correspondant.

9. Dispositif médical de perçage selon la revendication 8, **caractérisé en ce qu'**un dispositif de commutation est apte à être commandé par l'unité d'analyse pour arrêter l'entraînement de perçage ou pour émettre par l'intermédiaire du dispositif d'affichage (13) un signal optique ou acoustique correspondant, si l'unité d'analyse constate le perçage d'un type de tissu défini.

10. Dispositif médical de perçage selon l'une des revendications 1 à 9, **caractérisé en ce que** l'électrode de perçage (9) est en contact électrique avec le foret (7) au niveau de la zone annulaire (12).

11. Dispositif médical de perçage selon l'une des revendications 2 à 10, **caractérisé en ce que** le dispositif de mesure de profondeur de perçage (17) est couplé à un émetteur de signaux avec lequel le moment ou la position de départ au début de l'opération de mesure peut être signalé par l'utilisateur du dispositif de perçage au dispositif de mesure de profondeur de perçage (17).

12. Dispositif médical de perçage selon l'une des revendications 2 à 11, **caractérisé en ce que** grâce au dispositif de mesure de profondeur de perçage (17), l'avance de l'outil de perçage (7) peut être mesurée par rapport à un manchon de protection de tissu (6) qui est apte à être disposé à l'endroit où l'outil de perçage (7) pénètre dans le tissu à percer (2, 3, 4) et qui guide l'outil de perçage (7) à cet endroit.

13. Dispositif médical de perçage selon l'une des revendications 4 à 12, **caractérisé**
- **en ce qu'**il est prévu un dispositif de mesure de force de pression pour relever la force de pression avec laquelle un opérateur presse l'outil de perçage (7) contre le tissu à percer, et
- **en ce qu'**il est prévu un dispositif d'analyse de couple avec lequel les valeurs de couple mesurées par le dispositif de relevé de couple sont aptes à être normalisées suivant les valeurs de force de pression mesurées.

14. Dispositif médical de perçage selon l'une des revendications 4 à 13, **caractérisé en ce qu'**il est prévu un dispositif d'analyse de couple avec lequel peuvent être obtenues, sur la base des valeurs de mesure ou des variations de valeurs de mesure relevées par le dispositif de relevé de couple et/ou des valeurs de force de pression, des informations sur la qualité ou le type du tissu qui est percé par l'outil de perçage (7) au moment correspondant.

15. Dispositif médical de perçage selon la revendication 8 et l'une des revendications 4 à 14, **caractérisé en ce qu'**avec l'unité d'analyse peuvent être obtenues, grâce à une analyse combinée des valeurs de mesure ou des variations de valeurs de mesure relevées par le dispositif de mesure de résistance (11) et des valeurs de mesure ou des variations de valeurs de mesure relevées par le dispositif de relevé de couple, les informations sur la qualité ou le type du tissu qui est percé par l'outil de perçage (7) au moment correspondant.
